(19) Europäisches Patentamt
European Patent Office
Office européen des brevets

(11) Veröffentlichungsnummer: **0 006 149**
**B1**

(12) # EUROPÄISCHE PATENTSCHRIFT

(45) Veröffentlichungstag der Patentschrift: **06.01.82**

(51) Int. Cl.³: **C 07 D 307/68**

(21) Anmeldenummer: **79101617.3**

(22) Anmeldetag: **28.05.79**

(54) **Verfahren zur Herstellung von 2,5-Dimethylfuran-3-carbonsäurealkylestern.**

(30) Priorität: **14.06.78 DE 2826013**

(43) Veröffentlichungstag der Anmeldung: **09.01.80 Patentblatt 80/1**

(45) Bekanntmachung des Hinweises auf die Patenterteilung: **06.01.82 Patentblatt 82/1**

(84) Benannte Vertragsstaaten: **AT BE CH DE FR GB IT LU NL SE**

(56) Entgegenhaltungen:
**DE-A-1 935 009**
**DE-A-2 006 472**
**DE-A-2 207 098**
**DE-A-2 227 547**
**GB-A-1 387 652**
**US-A-4 025 537**

(73) Patentinhaber: **BASF Aktiengesellschaft, Carl-Bosch-Strasse 38, D-6700 Ludwigshafen (DE)**

(72) Erfinder: **Linhart, Friedrich, Dr., Leisberg 61, D-6900 Heidelberg (DE)**
Erfinder: **Girgensohn, Bjoern, Dr., Neckarpromenade 38, D-6800 Mannheim 1 (DE)**
Erfinder: **Merkle, Hans, Dr., Brahmsstrasse 4, D-6700 Ludwigshafen (DE)**
Erfinder: **Siegel, Hardo, Dr., Hans-Purrmann-Allee 25, D-6720 Speyer (DE)**
Erfinder: **Mueller, Hans-Richard, Dr., An der Tuchbleiche 9, D-6712 Bobenheim-Roxheim 2 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 006 149 B1

BUNDESDRUCKEREI BERLIN

# Verfahren zur Herstellung von 2,5-Dimethylfuran-3-carbonsäurealkylestern

Die vorliegende Erfindung betrifft ein Verfahren zur Herstellung von 2,5-Dimethylfuran-3-carbonsäureestern aus α-Acyloxypropionaldehyd und Acetessigester in Gegenwart von wasserfreiem Eisen(III)-chlorid.

2,5-Dimethylfuran-3-carbonsäurealkylester dienen als wertvolle Vorprodukte zur Herstellung von Pflanzenschutzmitteln. Es ist bekannt, 2,5-Dimethylfuran-3-carbonsäurealkylester durch Umsetzung von Acetessigsäurealkylestern mit α-Acetoxypropionaldehyd herzustellen (DE-A-2 207 098). Dieses Verfahren verläuft in zwei Stufen, wobei man nacheinander einen basischen und einen sauren Katalysator benötigt, so daß bei einer technischen Durchführung wegen der verschiedenen Empfindlichkeit der Reaktionsgefäße gegen saure und basische Reagenzien im Laufe des Verfahrens das Reaktionsgefäß gewechselt werden muß. Ferner muß das bei der Reaktion entstehende Wasser entweder durch Zugabe eines wasserentziehenden Mittels oder durch azeotrope Destillation entfernt werden.

Als saure Katalysatoren werden unter anderem auch Aluminiumchlorid und Zinkchlorid beschrieben.

Es ist ferner bekannt (DE-A-2 006 472), ähnliche Furancarbonsäuren durch Umsetzung eines α-Hydroxyketons mit Acetessigsäureäthylester herzustellen. Dieses Verfahren hat den Nachteil, daß das Lösungsmittel Benzol verwendet wird, das wegen seiner bekannten Toxizität nur unter strengen Sicherheitsvorkehrungen gehandhabt werden darf, und 2. pro 175 Gewichtsteilen Acetessigsäureäthylester 100 Gewichtsteile wasserfreies Zinkchlorid als Katalysator eingesetzt werden. Abgesehen von dem hohen Preis des Zinkchlorids verbietet sich aus Gründen des Umweltschutzes die Einleitung so großer Mengen dieser Buntmetallverbindung in das Abwasser.

Aus der DE-A-2 227 547 ist es bekannt, 2,5-Dimethylfuran-3-carbonsäureamide mit Hilfe eines sauren Katalysators herzustellen.

Es wurde nun gefunden, daß man 2,5-Dimethylfuran-3-carbonsäurealkylester der Formel

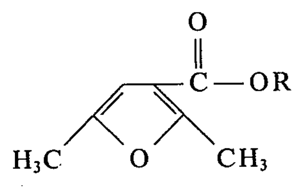

in der R einen niederen Alkylrest mit 1–4 C-Atomen, bevorzugt Äthyl oder Methyl, insbesondere Methyl, bedeutet, durch Umsetzung eines α-Acyloxypropionaldehyds der Formel

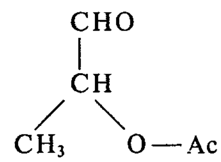

in der Ac einen Acylrest mit 2 bis 4 C-Atomen, vorzugsweise einen Acetylrest bedeutet, mit einem Acetessigester der Formel

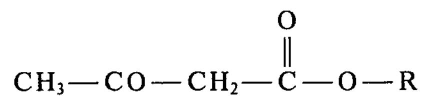

in der R die obengenannten Bedeutungen hat, bei einer Temperatur von +20 bis +130°C in einfacher Weise erhält, wenn man die Umsetzung in Gegenwart von festem wasserfreien Eisen(III)-chlorid durchführt. Hierbei setzt man zweckmäßig einer Lösung von Acetessigsäurealkylester in dem entsprechenden Alkohol nacheinander festes wasserfreies Eisen(III)-chlorid und α-Acetoxypropionaldehyd zu, wobei sich die Mischung erwärmt. Man hält sie noch einige Zeit im Temperaturbereich von 20 bis 130°C, vorzugsweise 40 bis 90°C, insbesondere 50 bis 80°C und trennt nach dem Verdünnen mit Wasser den abgeschiedenen Ester ab, wobei man diese Abtrennung durch Zugabe eines mit Wasser nicht mischbaren organischen Lösungsmittels, beispielsweise Toluol, Xylole, höhere alkylaromatische Verbindungen, chlorierte aromatische Verbindungen, z. B. Chlorbenzol, aliphatische Kohlenwasserstoffe, z. B. Cyclohexan, Hexan, Heptan, Oktan, Petroläther, Ligroin oder chlorierte aliphatische Kohlenwasserstoffe, z. B. Dichlormethan, Dichloräthan unterstützen kann, und destilliert das so erhaltene Rohprodukt.

Beispielsweise beträgt das Mengenverhältnis Eisen(III)-chlorid zu Acetessigester 0,25 : 1 bis 1,5 : 1 Gewichtsteile oder Eisen(III)-chlorid zu α-Acetoxypropionaldehyd 0,25 : 1 bis 1,5 : 1 Gewichtsteile und

das Gewichtsverhältnis Acetessigsäureäthylester zu $\alpha$-Acetoxypropionaldehyd 0,5 : 1 bis 1,5 : 1 Gewichtsteile.

Das beispielsweise bei der Reaktion

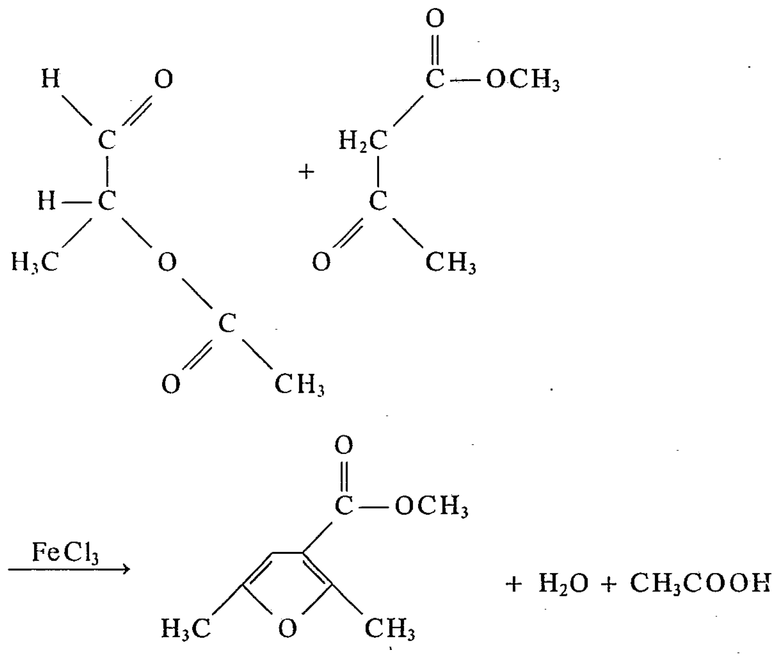


entstehende Wasser wird von dem Eisen(III)-chlorid, das zugleich als Katalysator wirkt, gebunden und der Reaktionsmischung entzogen. Das wasserfreie Eisen(III)-chlorid läßt sich an der Luft ohne besondere Vorsichts- und Sicherheitsmaßnahmen handhaben.

Da Eisensalze in den meisten natürlichen Gewässern, zum Beispiel den Eisensäuerlingen, und im Trinkwasser, wie sich zum Beispiel an der Abscheidung von Rost an Badewannen und anderen sanitären Einrichtungen beobachten läßt, vorkommen, ist die Einleitung von Eisen(III)-chlorid in das Abwasser absolut unproblematisch, bzw. sogar von Vorteil, da Eisen(III)-chlorid häufig in Kläranlagen als Flockungsmittel zugesetzt wird.

Die als Lösungsmittel verwendeten Alkohole sind billig und können wegen ihrer schnellen biologischen Abbaubarkeit ohne Bedenken ins Abwasser gebracht oder aber zurückgewonnen werden. Auch die bei der Reaktion entstehende Essigsäure wird von den Bakterien einer Kläranlage schnell zersetzt.

Das erfindungsgemäße Verfahren läßt sich in der Weise variieren, daß man das Reaktionsgemisch, anstatt mit Wasser zu zersetzen, einer Destillation unterwirft, wobei man entweder von Anfang an, oder aber erst bei der Destillation des gewünschten Esters, verminderten Druck anwendet. Auf diese Art kann auch das Lösungsmittel zurückgewonnen werden, während die im Reaktionsgefäß verbleibenden Eisensalze mit Wasser entfernt werden können.

Die folgenden Beispiele sollen die Vorteile des erfindungsgemäßen Verfahrens verdeutlichen:

## Beispiel 1

Zu einer Lösung von 34,8 Gewichtsteilen Acetessigsäuremethylester in 36 Gewichtsteilen Methanol gibt man 12,2 Gewichtsteile wasserfreies Eisen(III)-chlorid und anschließend 44,9 Gewichtsteile 85,5prozentigen (Gewichtsprozent) -Acetoxypropionaldehyd, wobei sich die Mischung bis zum Sieden erwärmt. Man erhitzt noch 1—6 Stunden unter Rückfluß, vermischt nach dem Abkühlen mit 80 Gewichtsteilen Wasser und mit 37,5 Gewichtsteilen Dichloräthan, trennt die Wasserphase ab und extrahiert sie noch zweimal mit 25 Gewichtsteilen Dichloräthan. Das Dichloräthan wird bei Normaldruck abdestilliert und anschließend wird im Vakuum das Produkt destilliert. Man erhält 39 Gewichtsteile 2,5-Dimethylfuran-3-carbonsäuremethylester vom Siedepunkt 92° C bei 26 mbar.

## Beispiel 2

Zu einer Lösung von 116 Gewichtsteilen Acetessigsäuremethylester in 120 Gewichtsteilen Methanol gibt man 27 Gewichtsteile wasserfreies Eisen(III)-chlorid und tropft anschließend so langsam 149 Gewichtsteile 85,5prozentigen $\alpha$-Acetoxypropionaldehyd zu, daß die Temperatur nicht über 37°C steigt. Nach dem Rühren über Nacht bei Raumtemperatur (20°C) erhitzt man 2 Stunden zum Sieden

und arbeitet nach dem Abkühlen auf, wie in Beispiel 1 beschrieben. Bei der Destillation erhält man 116,6 Gewichtsteile 2,5-Dimethylfuran-3-carbonsäuremethylester.

### Beispiel 3

Zu einer Lösung von 116 Gewichtsteilen Acetessigsäuremethylester in 120 Gewichtsteilen Methanol gibt man 27 Gewichtsteile Eisen(III)-chlorid und 149 Gewichtsteile 85,5prozentigen α-Acetoxypropionaldehyd, so daß sich die Mischung zum Sieden erwärmt. Man destilliert das Methanol bei Normaldruck ab und destilliert anschließend unter vermindertem Druck das Produkt. Man erhält 106,2 Gewichtsteile 2,5-Dimethylfuran-3-carbonsäuremethylester vom Siedepunkt 89° C bei 20 mbar.

### Beispiel 4

Zu einer Lösung von 130 Gewichtsteilen Acetessigsäureäthylester in 120 Gewichtsteilen Äthanol gibt man 27 Gewichtsteile wasserfreies Eisen(III)-chlorid und 149 Gewichtsteile 85,5prozentigen α-Acetoxypropionaldehyd, so daß sich die Mischung auf 80°C erwärmt. Das Äthanol wird bei Normaldruck abdestilliert. Anschließend destilliert man unter einem Druck von 25 mbar den 2,5-Dimethylfuran-3-carbonsäureäthylester bei der Siedetemperatur 96 – 100° C ab.

### Beispiel 5

Zu einer Lösung von 150 Gewichtsteilen Acetessigsäuremethylester in 120 Gewichtsteilen Methanol gibt man nacheinander 40 Gewichtsteile Eisen(III)-chlorid und 136 Gewichtsteile 85prozentigen α-Acetoxypropionaldehyd. Die sich erwärmende Mischung wird noch 2 Stunden unter Rückfluß erhitzt. Nach dem Abkühlen wäscht man die Reaktionslösung mit Wasser und destilliert. Man erhält 127 g 2,5-Dimethylfuran-3-carbonsäuremethylester.

**Patentansprüche**

1. Verfahren zur Herstellung von 2,5-Dimethylfuran-3-carbonsäurealkylestern der Formel

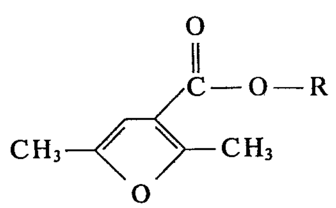

in der R einen niederen Alkylrest bedeutet durch Umsetzung eines α-Acyloxypropionaldehyds der Formel

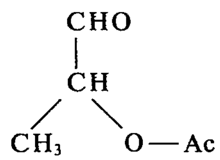

in der Ac einen Acylrest bedeutet, mit einem Acetessigester der Formel

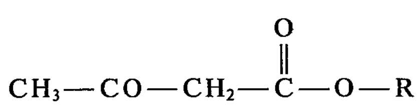

in der R die obengenannten Bedeutungen hat, bei einer Temperatur von +20 bis +130° C, dadurch gekennzeichnet, daß man die Umsetzung in Gegenwart von festem wasserfreien Eisen(III)-chlorid durchführt.

2. Verfahren nach Anspruch 1, zur Herstellung von 2,5-Dimethylfuran-3-carbonsäuremethylester, dadurch gekennzeichnet, daß man Acetessigsäuremethylester gelöst in Methanol und α-Acetoxypropionaldehyd als Ausgangsprodukte verwendet.

3. Verfahren nach Anspruch 1 zur Herstellung von 2,5-Dimethylfuran-3-carbonsäureäthylester,

dadurch gekennzeichnet, daß man Acetessigsäureäthylester gelöst in Äthanol und $\alpha$-Acetoxypropionaldehyd als Ausgangsprodukte verwendet.

**Claims**

1. A process for the preparation of a 2,5-dimethylfuran-3-carboxylic acid alkyl ester of the formula

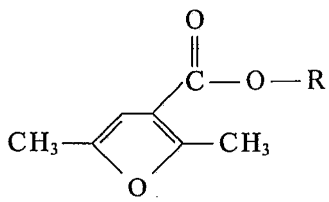

where R is lower alkyl, by reacting an $\alpha$-acyloxypropionaldehyde of the formula

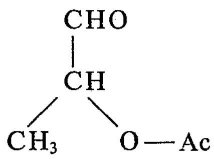

where Ac is acyl, with an acetoacetic acid ester of the formula

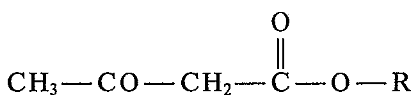

where R has the above meanings, at from +20 to +130°C, characterized in that the reaction is carried out in the presence of solid anhydrous iron-III chloride.

2. A process as claimed in claim 1 for the preparation of 2,5-dimethylfuran-3-carboxylic acid methyl ester, characterized in that methyl acetoacetate dissolved in methanol, and $\alpha$-acetoxypropionaldehyde are used as starting materials.

3. A process as claimed in claim 1 for the preparation of 2,5-dimethylfuran-3-carboxylic acid ethyl ester, characterized in that ethyl acetoacetate dissolved in ethanol, and $\alpha$-acetoxypropionaldehyde are used as starting materials.

**Revendications**

1. Procédé de préparation d'esters alkyliques d'acides 2,5-diméthylfuran-3-carboxyliques de formule

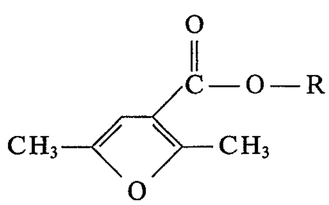

dans laquelle R représente un reste alkyle inférieur, par réaction, à une température de +20 à +130°C, d'un aldéhyde $\alpha$-acyloxy/propionique de formule

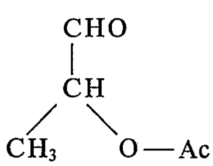

dans laquelle Ac représente un reste acyle, avec un ester acétylacétique de formule

$$CH_3-CO-CH_2-\overset{\overset{O}{\|}}{C}-O-R$$

dans laquelle R a la signification indiquée ci-dessus, caractérisé par le fait qu'on effectue la réaction en présence de chlorure ferrique, solide et exempt d'eau.

2. Procédé selon la revendication 1, pour la préparation d'ester méthylique d'acide 2,5-di-méthylfuran-3-carboxylique, caractérisé par le fait qu'on utilise, comme produits de départ, de l'ester méthylique d'acide acétylacétique dissous dans du méthanol et de l'aldéhyde $\alpha$-acétoxypropionique.

3. Procédé selon la revendication 1, pour la préparation d'ester éthylique d'acide 2,5-di-méthylfuran-3-carboxylique, caractérisé par le fait qu'on utilise, comme produits de départ, de l'ester éthylique d'acide acétylacétique dissous dans de l'éthanol et de l'aldéhyde $\alpha$-acétoxypropionique.